Europäisches Patentamt

European Patent Office

Office européen des brevets

(11) Veröffentlichungsnummer: **0 474 994 A1**

## (12) EUROPÄISCHE PATENTANMELDUNG

(21) Anmeldenummer: **91110717.5**

(22) Anmeldetag: **28.06.91**

(51) Int. Cl.5: **A61N 5/10**, G21F 5/015

(30) Priorität: **08.09.90 DE 4028625**

(43) Veröffentlichungstag der Anmeldung:
**18.03.92 Patentblatt 92/12**

(84) Benannte Vertragsstaaten:
**FR GB NL**

(71) Anmelder: **ISOTOPEN-TECHNIK DR. SAUERWEIN GMBH**
**Bergische Strasse 16**
**W-5657 Haan/Rheinl. 1(DE)**

(72) Erfinder: **Rohe, Karl-Heinz**
**Ohligser Strasse 85**
**W-5657 Haan 1(DE)**

Erfinder: **Weinlich, Karl**
**Weberstrasse 18**
**W-5600 Wuppertal 2(DE)**
Erfinder: **Bormann, Ulrich**
**Hagmanngarten 1**
**W-4300 Essen-Heisingen 15(DE)**
Erfinder: **Link, Rainer, Dr.**
**Buchenhöhe 1**
**W-5014 Kerpen(DE)**

(74) Vertreter: **König, Reimar, Dr.-Ing. et al**
**Patentanwälte Dr.-Ing. Reimar König**
**Dipl.-Ing. Klaus Bergen Wilhelm-Tell-Strasse**
**14 Postfach 260162**
**W-4000 Düsseldorf 1(DE)**

(54) **Verfahren und Vorrichtung zum Ein- und Zurückfahren einer radioakfiven Strahlenquelle in einem Applikator.**

(57) Verfahren und Vorrichtung zum Einfahren radioaktiver Strahlenquellen in Applikatoren und zum Zurückfahren der Strahlenquelle und Ablegen in mindestens einer abgeschirmten Belade- und/oder Lagerstation. Zum Entnehmen einer Strahlenquelle (40) aus der Belade- und/oder Lagerstation (4) wird ein Zugkabel (33, 46) mit einer in Zugrichtung wirksamen Kupplung (35) über eine Gabelung (20) und eine Weiche (17) in die Belade- und/oder Lagerstation vorgefahren, mit der Strahlenquelle gekuppelt, bis zu einem Anschlag zurückgefahren, dort entkuppelt und das Zugkabel über die Gabelung zurückgefahren. Zum Einbringen der Strahlenquelle in den Applikator (12) wird hingegen ein Schubkabel (36) mit einer nur in Schubrichtung wirksamen Kupplung (38, 39) über die Gabelung an die Strahlenquelle herangefahren und unter Mitnahme dieser Strahlenquelle über die Weiche bis zum Applikator vor- und von dort wieder zurückgefahren.

Fig. 1

Die Erfindung betrifft ein Verfahren und eine Vorrichtung zum Einfahren radioaktiver Strahlenquellen in Applikatoren und zum Zurückfahren der Strahlenquellen, mit mindestens einer abgeschirmten Belade- und/oder Lagerstation für die Strahlenquellen, einem flexiblen, in einem Kanal geführten Schubkabel mit einer die Strahlenquelle in der Bestrahlungsposition freigebenden Kupplung.

Eine derartige Vorrichtung ist in der europäischen Offenlegungsschrift 0 158 630 beschrieben; sie besteht aus einem oder mehreren identischen Modulen aus je einer abgeschirmten Belade- und/oder Lagerstation für gegebenenfalls unterschiedliche Strahlenquellen sowie je eine Schubstange und bietet die Möglichkeit, in der Bestrahlungsposition die Fördervorrichtung von einer Hohlnadel zu trennen, indem zwischen der Strahlenquelle und der Schubstange eine in der Bestrahlungsposition lösbare Kupplung betätigt wird. Die Strahlenquelle läßt sich mit der Schubstange fernbedient in die Hohlnadel einbringen, wird dort freigegeben und abgelegt. Während der Behandlung kann die Hohlnadel von der Schubstange getrennt werden, so daß der Patient weitgehende Bewegungsfreiheit erhält. Nach Beendigung der Behandlung wird die Fördervorrichtung wieder mit der Hohlnadel gekuppelt, so daß sich die einzelnen Strahlenquellen nach Einrasten der Kupplung einzeln wieder in die Belade- und/oder Lagerstation zurückholen lassen.

Nachteilig ist bei der bekannten Vorrichtung daß für jede Strahlenquelle ein Modul mit einer Fördervorrichtung erforderlich ist. Gravierender ist noch, daß die Kupplung im jeweiligen Applikator gelöst werden muß, um jeweils eine Strahlenquelle im Applikator zu belassen. Da jedoch die Platzverhältnisse in interstitiell eingebrachten, hohlen Bestrahlungsnadeln sehr beengt sind, ist es schwierig, einen sicher arbeitenden Lösemechanimus für die Kupplung in der Bestrahlungsnadel anzuordnen.

Der Erfindung liegt daher die Aufgabe zugrunde, eine Vorrichtung und ein Verfahren zu schaffen, mit denen es möglich ist, Strahlenquellen von einer Belade- und/oder Lagerstation in einen Applikator zu bringen, dort von der Fördervorrichtung zu trennen und für die Dauer der Behandlung eines Patienten zu belassen sowie nach Beendigung der Behandlung mittels der Fördervorrichtung zurückzuholen, ohne die Kupplung im Bereich des Applikators zu betätigen oder anzusteuern. Die dafür eingesetzten Mittel sollten einfach aufgebaut und leicht zu bedienen sein.

Ausgehend von dieser Aufgabenstellung besteht die Erfindung darin, daß bei einer Vorrichtung der eingangs erwähnten Art erfindungsgemäß zwei Antriebe mit jeweils einem in einem Kanal geführten Schub- bzw. Zugkabel verbunden und die Kanäle einer Gabelung zusammengeführt sind, daß das Zugkabel im Zusammenwirken mit einer Strahlequelle eine in Zugrichtung wirksame lösbare Kupplung und das Schubkabel eine nur in Schubrichtung wirksame lösbare Kupplung aufweist, daß eine Weiche zwischen den zu den Applikatoren und den zu der Belade- und/oder Lagerstation führenden Kanälen einerseits sowie der Gabelung andererseits angeordnet ist und sich zwischen der Weiche und der Gabelung ein Auslöser für die in Zugrichtung wirksame Kupplung befindet.

Die Weiche kann mehrere Eingänge und Ausgänge aufweisen. Die Eingänge können über Kanäle mit der Belade- und/oder Lagerstation und die Ausgänge über entsprechende Kanäle mit einer entsprechenden Anzahl von Applikatoren verbunden sein. Um eine Strahlenquelle aus einem Lagerkanal im Belade- und/oder Lagerbehälter herauszuholen und in einen der Applikatoren einzubringen, wird zunächst das eine in Zugrichtung wirksame lösbare Kupplung aufweisende Zugkabel mittels einer der beiden Antriebe über die Gabelung und die entsprechend geschaltete Weiche in den Lagerkanal der Belade- und/oder Lagerstation verfahren, mit der Strahlenquelle gekuppelt und zieht diese bis in den Bereich des zwischen der Gabelung und der Weiche angeordneten Auslösers für die in Zugrichtung wirksame Kupplung zurück. Dort wird die Strahlenquelle vom Zugkabel getrennt, das Zugkabel weiter bis in seine Endstellung zurückgefahren, und das Schubkabel durch die Gabelung hindurch bis zur Strahlenquelle gefahren; es schiebt die Strahlenquelle über die Weiche bis in den Applikator. Da zwischen diesem Schubkabel und der Strahlenquelle keinerlei in Zugrichtung wirksame Verbindung besteht, sondern das Schubkabel die Strahlenquelle nur durch Schieben vorwärtsbewegt, läßt sich dieses Schubkabel nach Verbringen der Strahlenquelle in den Applikator in seine Ausgangsstellung zurückziehen, während die Strahlenquelle im Applikator verbleibt.

Es ergibt sich somit, daß sich mit zwei Antrieben, von denen der eine die Strahlenquelle in Zugrichtung und der andere in Schubrichtung bewegt und durch die Anordnung einer Gabelung und einer Weiche eine sehr große Anzahl von Strahlenquellen aus einer Belade- und/oder Lagerstation in eine entsprechende Zahl von Applikatoren verbringen läßt. Danach können die Applikatoren von der Vorrichtung getrennt werden und am Patienten verbleiben. Erst nach Beendigung der Behandlung muß die Vorrichtung wieder mit den Applikatoren gekuppelt werden, und die Strahlenquellen werden nacheinander in der beschriebenen Weise in die Belade- und/oder Lagerstation zurückbefördert.

Die Strahlenquellen können aus nadelförmigen, mit radioaktivem Material gefüllten Haltern bestehen, an deren einem Ende eine Hülse und nach innen gerichtete Federelemente angeordnet sind, die mit einem an einem Zugkabelende angeordneten, in die Hülse einführbaren Stift mit einem mit den Federenden zusammenwirkenden Durchmesser die in Zugrichtung wirksame lösbare Kupplung bildet.

Der in die Hülse einführbare Stift wird durch die Federenden mittels Reibung gehalten und läßt sich auf einfache Weise mittels eines mit einer Stirnseite der Hülse zusammenwirkenden Anschlags trennen. Hierzu kann der größte Durchmesser des Zugkabelendes mit dem Stift kleiner sein als der Durchmesser der Hülse und der Anschlag aus einer Kabeldurchführung mit einem Innendurchmesser größer als der Durchmesser des Zugkabelendes mit dem Stift aber kleiner als der Hülsendurchmesser bestehen.

Die Strahlenquellen können auch aus nadelförmigen, mit radioaktivem Material gefüllten Haltern bestehen, an deren einem Ende eine Hülse mit federnd nach innen gerichteten Haken angeordnet ist, die mit einem an einem Zugkabelende angeordneten Stift mit einer Rastnut für die Haken die in Zugrichtung wirksame lösbare Kupplung bilden. In diesem Fall ergibt sich eine formschlüssige Verbindung zwischen den Haken und der Rastnut im Stift, die sich während des Transports der Strahlenquelle durch die Kanäle nicht lösen kann. In diesem Fall kann der Auslöser für die in Zugrichtung wirksame Kupplung aus Schrägflächen an die Haken untergreifenden Keilflächen bestehen, wobei der größte Durchmesser des Zugkabelendes mit dem Stift kleiner ist als der Durchmesser der Hülse und der Innendurchmesser einer Kabeldurchführung durch den Auslöser größer als der Durchmesser des Zugkabelendes mit Stift aber kleiner als der Hülsendurchmesser ist.

Des weiteren können die Strahlenquellen aus nadelförmigen, mit radioaktivem Material gefüllten Haltern bestehen, deren eines Ende aus magnetischem Material besteht, das mit einem an einem Zugkabelende angeordneten Ansatz aus magnetischem Material die in Zugrichtung wirksame lösbare Kupplung bildet. In diesem Fall kann der Auslöser in gleicher Weise aufgebaut sein wie oben beschrieben und aus einem mit einer Stirnseite des Halterendes zusammenwirkenden Anschlag bestehen.

Die Erfindung wird nachstehend anhand mehrerer in der Zeichnung dargestellter Ausführungsbeispiele des näheren erläutert. In der Zeichnung zeigen:

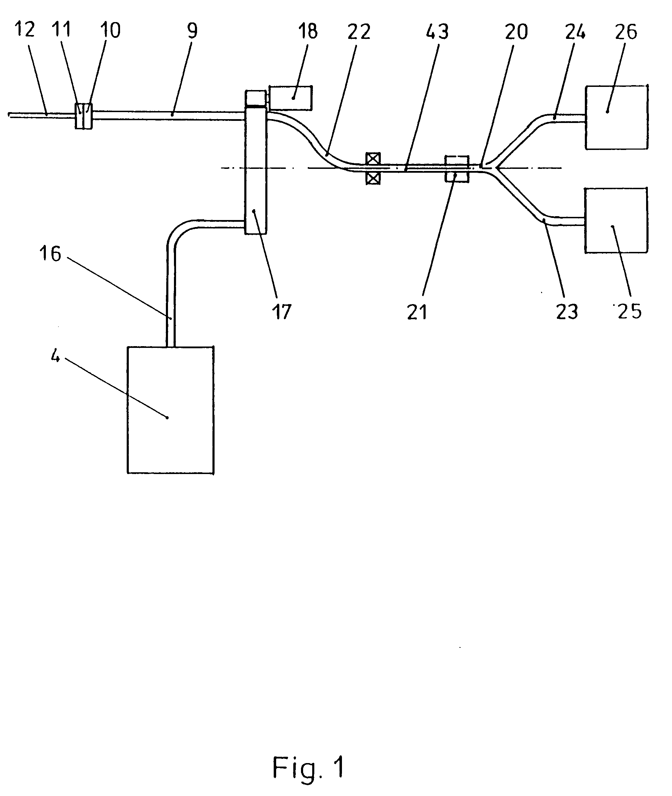

Fig. 1    die schematische Darstellung einer erfindungsgemäßen Vorrichtung,

Fig. 2    die Ansicht eines Zugkabelendes mit einem in Zugrichtung wirksamen Kupplungsteil, teilweise im Schnitt,

Fig. 3    ein Schubkabelende mit einer nur in Schubrichtung wirksamen Kupplung,

Fig. 4    das Ende eines nadelförmigen Halters mit einer in Zugrichtung wirksamen Kupplung, teilweise im Schnitt,

Fig. 5    eine andere in Zugrichtung wirksame Kupplung mit einem entsprechenden Auslöser,

Fig. 6    eine seitliche Schnittansicht einer erfindungsgemäßen Vorrichtung und

Fig. 7    einen Horizontalschnitt der Vorrichtung gemäß Fig. 6.

Die erfindungsgemäße Vorrichtung besteht in ihrer einfachsten Form aus einem abgeschirmten Belade- und/oder Lagerbehälter 4 für im einzelnen nicht dargestellte Strahlenquellen. Dieser Behälter 4 ist über einen Kanal 16 mit einer Weiche 17 verbunden, die sich mittels eines Antriebsmotors 18 verstellen läßt. Eine derartige Weiche ist im einzelnen in der europäischen Patentschrift 0 128 300 beschrieben. Von der Weiche 17 führt ein weiterer Kanal 9 über eine Kupplung 10 und eine Anschlußplatte 11 zu einem Applikator 12. Die von den Kanälen 9 und 16 abgewandte Seite der Weiche 17 ist über einen drehbaren Kanal 22 und ein kurzes Kanalstück 43 sowie einen Auslöser 21 mit einer Gabelung 20 verbunden, deren Kanäle 23, 24 zu entsprechenden Antrieben 25, 26 führen. Derartige Antriebe sind zum Beispiel in der deutschen Patentschrift 33 35 438 im einzelnen beschrieben. Mittels der Antriebe 25, 26 lassen sich nicht dargestellte Schub- bzw. Zugkabel durch die Kanäle 23, 24, 43 sowie den drehbaren Kanal 22 entweder über den Kanal 16 in den Belade- und/oder Lagerbehälter 4 oder über den Kanal 9 bis zum Applikator 12 führen.

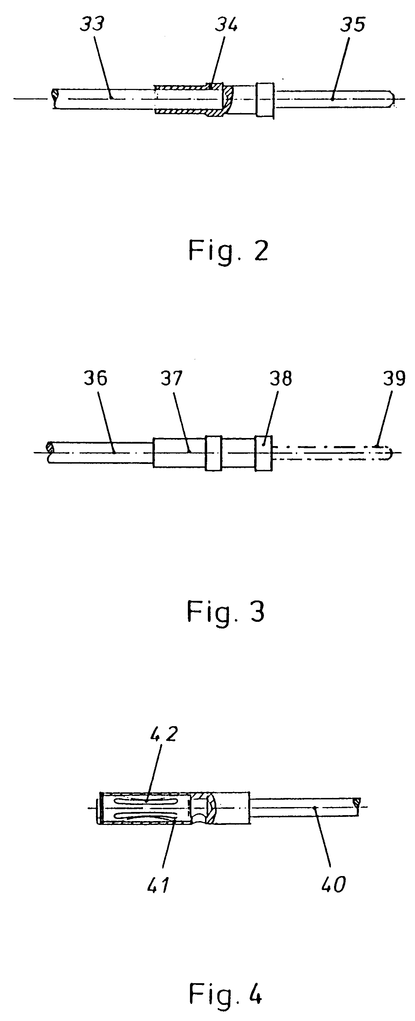

Das von dem Antrieb 25 bewegte Zugkabel 33 weist, wie in Fig. 2 dargestellt, an seinem Ende eine Muffe 34 mit einem geringfügig größeren Durchmesser als das Zugkabel 33 auf und setzt sich in einem Stift 35 mit kleinerem Durchmesser fort. Ein nadelförmiger Halter 40 (Fig. 4), der mit radioaktivem Material gefüllt ist, bildet die vom Belade- und/oder Lagerbehälter 4 in den Applikator 12 zu verbringende Strahlenquelle. Am Ende des nadelförmigen Halters 40 befindet sich eine Hülse 41 mit nach innen gerichteten Federelementen 42. Der Außendurchmesser des Stiftes 35 ist auf den Innendurchmesser der Hülse 41 so abgestimmt, daß beim Einschieben des Stiftes 35 in die Hülse 41 mittels der Federelemente 42 eine Klemmverbindung entsteht.

Wird somit das Zugkabel 33 mittels des Antriebs 25 bei entsprechend gestellter Weiche 17 bis in den Belade- und/oder Lagerbehälter 4 vorgefahren, schiebt sich der Stift 35 in die Hülse 41 hinein, da der nadelförmige Halter 40 in seiner Endstellung steht. Wird die Arbeitsrichtung des Antriebs 25 umgekehrt, läßt sich der nadelförmige Halter 40 mittels des Zugkabels 33 zurückziehen bis er in den Bereich des Auslösers 21 gelangt. Der Auslöser 21 besteht in einfachster Weise aus einer Durchmesserverengung vor der Gabelung 20, durch die sich das Kabel 33 mit der Muffe 34 und dem Stift 35 hindurchführen läßt, während der Durchmesser der Hülse 41 so groß ist, daß sie an dem Auslöser 21 anstößt. Der nadelförmige Halter 40 wird daher an dieser Stelle zwangsläufig vom Zugkabel 33 getrennt. Danach wird der Antrieb 26 eingeschaltet, und das entsprechende Schubkabel 36 (Fig. 3) schiebt sich mit seiner Muffe 37 und einem zylindrischen Ansatz 38 gegen die Hülse 41. Am zylindrischen Ansatz 38 kann ebenfalls ein Stift 39 angeordnet sein, der jedoch einen so kleinen Durchmesser besitzt, daß er durch die nach innen gerichteten Federelemente 42 der Hülse 41 nicht eingeklemmt wird, um zu vermeiden, daß auf diese Weise wieder eine in Zugrichtung wirkende Kupplung entsteht. Mittels des Schubkabels 36 läßt sich der nadelförmige Halter 40 nach Umschalten der Weiche 17 mit dem drehbaren Kanal 22 in den Applikator 12 vorbewegen. Wenn das Schubkabel 36 nunmehr in die Ausgangsstellung zurückbewegt wird, verbleibt der nadelförmige Halter 40 im Applikator 12, so daß sich die Applikatorkupplung 10 problemlos lösen läßt und der Patient mit dem Applikator 12 eine erheblich verbesserte Bewegungsfreiheit erhält.

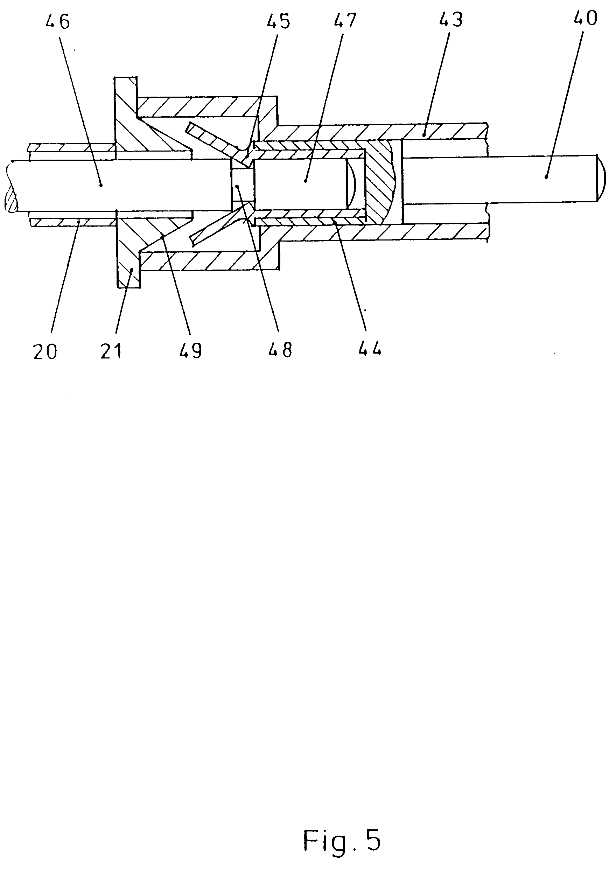

In Fig. 5 ist eine formschlüssige, in Zugrichtung wirksame Kupplung, zusammen mit dem zugehörigen Auslöser 21 dargestellt. Der nadelförmige Halter 40 weist in diesem Fall eine geschlitzte Hülse 44 auf, in deren Schlitze federnde Haken 45 angeordnet sind. Die federnden Haken wirken mit einer Rastnut 48 in einem Stift 47 am Ende eines Zugkabels 46 zusammen. Fig. 5 zeigt, daß sich der Stift 47 zwischen die federnden Haken 45 in der Hülse 44 einschieben läßt, nach Einrasten der federnden Haken 45 in die Rastnut 48 jedoch in Zugrichtung fest mit dem Zugkabel 46 verbunden ist. Zum Lösen des nadelförmigen Halters 40 vom Zugkabel 46 wird das Zugkabel 46 soweit zurückgezogen, daß Schrägflächen an den federnden Haken 45 in den Bereich von Keilflächen 49 an dem Auslöser 21 gelangen. Dadurch werden die federnden Haken 45 aufgeweitet, treten aus der Rastnut 48 aus, und das Zugkabel 46 läßt sich von dem nadelförmigen Halter 40 lösen. Ein Kanal 43 am Auslöser 21 bietet genügend Raum für das Auseinanderbewegen der federnden Haken 45 im Bereich des Auslösers 21, gewährt im übrigen Bereich jedoch eine genaue Führung des nadelförmigen Halters 40.

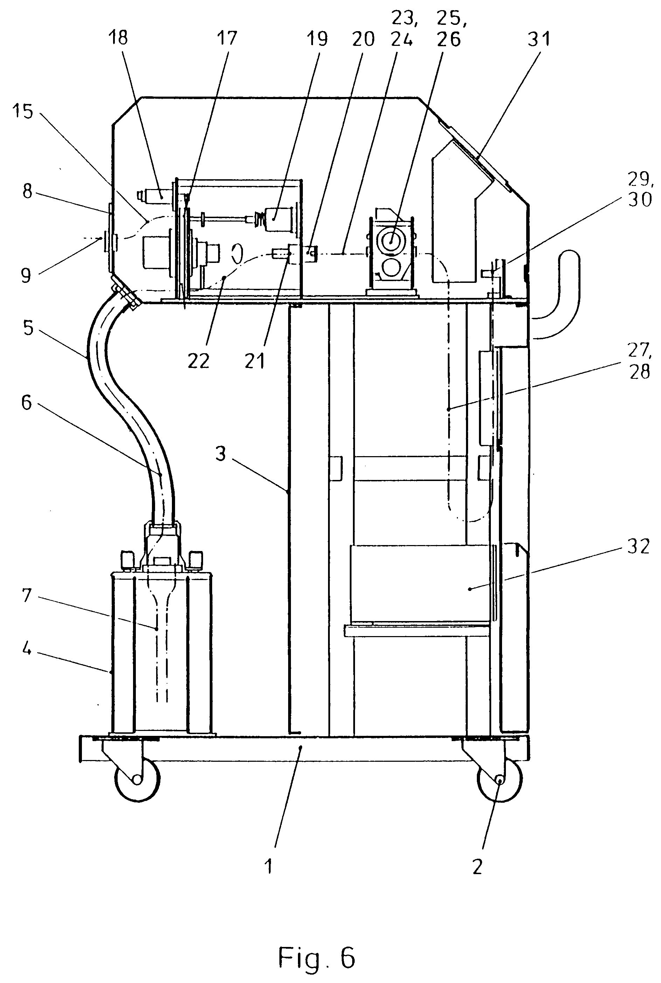

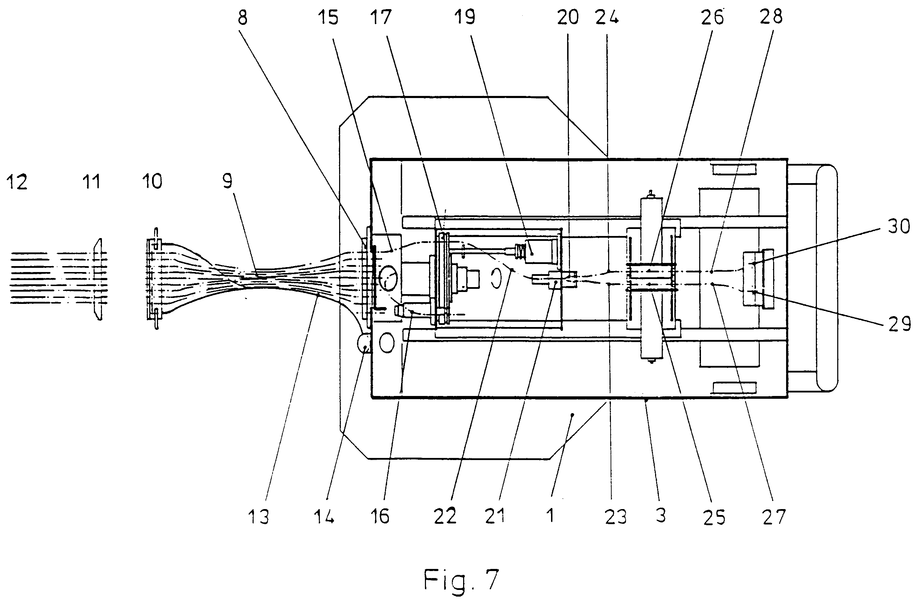

Die Fig. 6 und 7 enthalten eine detailliertere Darstellung der erfindungsgemäßen Vorrichtung. Auf einem Fahrwerk 1 mit Rollen 2 ist ein Gehäuse 3 angeordnet, das auf dem Fahrwerk 1 Platz für einen abgeschirmten Belade- und/oder Lagerbehälter 4 freiläßt. Der Belade- und/oder Lagerbehälter 4 ist über ein Rohr 5 mit dem Gehäuse 3 verbunden. Der Belade- und/oder Lagerbehälter 4 weist zum Beispiel 16 Lagerkanäle 7 für Strahlenquellen in Form der in Fig. 4 dargestellten nadelförmigen Halter 40 auf. Innerhalb des Rohres 5 sind dementsprechend sechzehn Kabelkanälen 6 zu der schaltbaren Weiche 17 geführt. Von derselben Seite der Weiche 17 führen Kanäle 15 zu einer Anschlußplatte 8 für ebenfalls sechzehn Kabelkanäle 9. Diese Kabelkanäle 9 enden in der Applikatorkupplung 10, die sich mit der Anschlußplatte 11 für die Applikatorkanäle kuppeln läßt. Mit dieser Anschlußplatte 11 sind sechzehn Applikatoren 12 in Form von Nadeln verbunden.

Die Kupplung 10 weist eine optische Überwachung des einwandfreien Anschlusses der Anschlußplatte 11 an die Kupplung 10 in Form eines Lichtleiters 13 auf, der von der Kupplung 10 zu einem Lichtleiteranschluß 14 geführt ist. Dies verhindert, daß Schubkabel bzw. Zugkabel und/oder Strahlenquellen aus der Kupplung 10 herausgefahren werden, wenn diese nicht mit der Anschlußplatte 11 verbunden ist.

Um den drehbaren Kanal 22 an der Weiche 17 bezüglich der Anschlüsse für die Kanäle 6 und 15 genau zu positionieren, rastet in jeder angesteuerten Stellung eine Verriegelung 19 in die Weiche 17 ein. Vor jeder Weichendrehung wird mittels eines mechanischen Tasters geprüft, ob kein Kabel oder Strahlerhalter in der Weiche liegt.

Da die Kabelkanäle zu dem zu behandelnden Patienten einen bis zwei Meter lang sein können, müssen auch die durch die Antriebe 25, 26 bewegten Schub- bzw. Zugkabel entsprechend lang sein, so daß auf der Rückseite der Antriebe 25, 26 Speicherkanäle 27, 28 angeordnet sind, in die die Schub- bzw. Zugkabel geführt werden, bis sie auf Endschalter 29, 30 treffen, die die Antriebe 25, 26 abschalten.

Die Vorrichtung ist in Form eines Pultes aufgebaut und weist eine Frontplatte 31 mit LCD-Anzeige und Tastern auf. Im unteren Teil des Gehäuses 3 sind ein Behälter 32 mit einem Akkumulator und eine Steuerelektronik untergebracht.

Mit dem erfindungsgemäßen Gerät lassen sich Strahlenquellen programmiert in Applikatoren einbringen und zurückholen, ohne daß das Bedienungspersonal radioaktiver Strahlung ausgesetzt ist.

**Patentansprüche**

1. Vorrichtung zum Einfahren radioaktiver Strahlenquellen in Applikatoren und zum Zurückfahren der Strahlenquellen, mit mindestens einer abgeschirmten Belade- und/oder Lagerstation für die Strahlenquellen, einem flexiblen, in einem Kanal geführten Schubkabel mit einer die Strahlenquelle in der Bestrahlungsposition freigebenden Kupplung, dadurch gekennzeichnet, daß zwei Antriebe (25, 26) mit jeweils einem in einem Kanal (23, 24) geführten Schubkabel bzw. Zugkabel (33, 36) verbunden und die Kanäle in einer Gabelung (20) zusammengeführt sind, daß das Zugkabel (33, 46) im Zusammenwirken mit einer Strahlenquelle (40) eine in Zugrichtung wirksame lösbare Kupplung (35, 41, 42; 47, 48; 44, 45) und das Schubkabel (36) eine nur in Schubrichtung wirksame lösbare Kupplung (38, 39) aufweist, daß eine Weiche (17) zwischen den zu den Applikatoren (12) und den zu der Belade- und/oder Lagerstation (4) führenden Kanälen (9,16) einerseits sowie der Gabelung (20) andererseits angeordnet ist und sich zwischen der Weiche und der Gabelung ein Auslöser (21) für die in Zugrichtung wirksame Kupplung (35, 41, 42; 44, 45, 47, 48) befindet.

2. Vorrichtung nach Anspruch 1, dadurch gekennzeichnet, daß die Strahlenquellen (40) aus nadelförmigen, mit radioaktivem Material gefüllten Haltern (40) bestehen, an deren einem Ende eine Hülse (41) mit nach innen gerichteten Federelementen (42) angeordnet ist, die mit einem an einem Zugkabelende (33) angeordneten, in die Hülse (41) einführbaren Stift (35) mit einem mit den Federenden zusammenwirkenden Durchmesser die in Zugrichtung wirksame lösbare Kupplung bildet.

3. Vorrichtung nach Anspruch 2, dadurch gekennzeichnet, daß der Auslöser für die in Zugrichtung wirksame Kupplung (21) aus einem mit einer Stirnseite der Hülse (41) zusammenwirkenden Anschlag bestehen.

4. Vorrichtung nach Anspruch 3, dadurch gekennzeichnet, daß der größte Durchmesser des Zugkabelendes (33, 34) mit dem Stift (35) kleiner ist als der Durchmesser der Hülse (41) und der Anschlag aus einer Kabeldurchführung mit einem Innendurchmesser größer als der Durchmesser des Zugkabelendes (33, 34) mit dem Stift (35) aber kleiner als der Hülsendurchmesser besteht.

5. Vorrichtung nach Anspruch 1, dadurch gekennzeichnet, daß die Strahlenquellen aus nadelförmigen, mit radioaktiven Material gefüllten Haltern (40) bestehen, an deren einem Ende eine Hülse (44) mit federnd nach innen gerichteten Haken (45) angeordnet ist, die mit einem an einem Zugkabelende angeordneten Stift (47) mit einer Rastnut (48) für die Haken (45) die in Zugrichtung wirksame lösbare Kupplung bilden.

6. Vorrichtung nach Anspruch 5, dadurch gekennzeichnet, daß der Auslöser für die in Zugrichtung wirksame Kupplung aus Schrägflächen an die Haken (45) untergreifenden Keilflächen (49) besteht, daß der größte Durchmesser des Zugkabelendes mit dem Stift (47) kleiner ist als der Durchmesser der Hülse (44) und der Innendurchmesser einer Kabeldurchführung durch den Auslöser (21) größer als der Durchmesser des Zugkabelendes mit dem Stift (47) aber kleiner als der Hülsendurchmesser ist.

7. Vorrichtung nach Anspruch 1, dadurch gekennzeichnet, daß die Strahlenquellen aus nadelförmigen, mit radioaktivem Material gefüllten Haltern (40) bestehen, deren eines Ende aus einem magnetischem Material besteht, das mit einem an einem Zugkabelende angeordneten Ansatz aus magnetischem Material die in Zugrichtung wirksame lösbare Kupplung bildet.

8. Vorrichtung nach Anspruch 7, dadurch gekennzeichnet, daß der Auslöser für die in Zugrichtung wirksame Kupplung aus einem mit einer Stirnseite des Halterendes zusammenwirkenden Anschlag besteht.

Fig. 1

33    34    35

## Fig. 2

36    37    38    39

## Fig. 3

42

41    40

## Fig. 4

46    45  47    43    40

20    21    49    48    44

Fig. 5

Fig. 6

Fig. 7

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5 ) |
|---|---|---|---|
| A | EP-A-0 152 124 (E. VAN 'T HOOFT)  * das ganze Dokument * | 1,2,5 | A61N5/10 G21F5/015 |
| A,D | EP-A-0 138 088 (K. SAUERWEIN)  * das ganze Dokument * | 1,2,5 | |
| A | GB-A-1 027 078 (T.E.M INTRUMENTS LIM.) 20. April 1966  * das ganze Dokument * | 1-3,5 | |
| A | US-A-4 943 731 (G.A. BROWN)  * das ganze Dokument * | 1,2 | |
| A,D | EP-A-0 158 630 (ÖSTERREICHES FORSCHUNGSZENTRUM SEIBERSDORF GES.M.B.H.)  * das ganze Dokument * | 1,2 | |
| A | EP-A-0 308 630 (K. SAUERWEIN)  * das ganze Dokument * | 7 | |
| | ----- | | **RECHERCHIERTE SACHGEBIETE (Int. Cl.5 )**  A61N G21F A61M |

Der vorliegende Recherchenbericht wurde für alle Patentansprüche erstellt

| Recherchenort | Abschlußdatum der Recherche | Prüfer |
|---|---|---|
| DEN HAAG | 18 NOVEMBER 1991 | FERRIGNO A. |